# EUROPÄISCHE PATENTANMELDUNG

(11) **EP 2 113 194 A1**
(43) Veröffentlichungstag der Anmeldung: **04.11.2009**
(21) Anmeldenummer: 08155570.8
(22) Anmeldetag: 02.05.2008
(51) Int. Cl.: A61B 5/00, A61B 5/04

(54) **Vorrichtung und Verfahren zur akustischen und visuellen Darstellung von aufbereiteten physiologische Daten und Verwendung der aufbereiteten Daten**

(71) Anmelder: Paracelsus Klinik Lustmühle AG, 8062 Lustmühle (CH)
(72) Erfinder: Falkner, Michael, 75446 Wiernsheim (DE)

(57) **Zusammenfassung**

Die Erfindung betrifft eine Vorrichtung (10) und ein Verfahren zum Auswerten von physiologische Daten. Die Vorrichtung (10) umfasst dabei wenigstens einen Sensor (20) zur Aufnahme der physiologische Daten, eine Betriebssoftware (50) zur Auswertung der physiologische Daten und zur Zuordnung der ausgewerteten physiologische Daten zu einem Steuer-Signal und wenigstens eine Ausgabeeinheit (34 a,b,c) für das Steuer-Signal.

Erfindungsgemäss ist vorgesehen, dass eine Speichereinheit für eine Vielzahl akustischer und/oder visueller Signalabfolgen aufweist. Erfindungsgemäss ist ferner vorgesehen, dass eine Zuordnung wenigstens einer akustischen und/oder visuellen Signalabfolge zu den ausgewerteten physiologische Daten, bevorzugt in Echtzeit, erfolgt.

## Beschreibung

### Definition von Begriffen

Für die nachfolgende Beschreibung sowie den Ansprüchen und den Zeichnungen werden Grundbegriffe verwendet, die zur Klarstellung nachfolgend erläutert werden.

### Biofeedback-Verfahren

Das Biofeedback-Verfahren ist aus dem Bereich der psychosomatischen Forschung der Verhaltenstherapie bekannt. Es dient dazu, biologische Veränderungen am menschlichen Körper, die nicht unmittelbar durch die Sinneswahrnehmung zugänglich sind, durch technische, insbesondere elektronischen Hilfsmitteln, beobachtbar zu machen, um dem Nutzer des Biofeedback-Verfahrens die Zustandsgrössen visuell wahrnehmbar zu machen. Somit können physiologische Messungen, wie beispielsweise Puls, Hautleitwerte oder Gehirnströme, visuell oder auch akustisch dargestellt werden, um so dem Nutzer des Biofeedback-Verfahrens zum einen eine Rückkopplung zu geben und zum anderen in einem weiteren Schritt eine Regulation zu ermöglichen. Somit kann der Nutzer aktiv beispielsweise Atemfrequenz, Blutdruck, Hautleitwert, Pulsfrequenz oder ähnliches ändern, so dass dies wiederum einen Einfluss auf die physiologischen Messgrössen hat.

Ziel der Anwendung von Biofeedback- und anderen psychoregulativen Verfahren ist es, eine psychische Stabilität und eine Balance des Nutzers zu erreichen. Der Nutzer soll in der Lage sein, einen Aktivierungszustand herbeizuführen und somit sein Potenzial voll ausschöpfen zu können. Befindet sich der Nutzer in einem ausgeglichenen und ausbalancierten Zustand, so kann er leistungshindernde Faktoren erkennen und verändern. Die Befähigung dafür kann, insbesondere durch ein Biofeedback- und andere Anwendungen des Biofeedback-Verfahrens bereitgestellt werden. Das Biofeedback-Verfahren kann auch als psychoregulatives Verfahren zur Leistungsoptimierung von jeglichen Personen eingesetzt werden, die beispielsweise im Arbeitsleben oder im Bereich des Sports leistungsorientiert tätig sind.

### Physiologische Parameter

Physiologische Parameter sind messbare körpereigene Signale, die mit psychischen, mentalen Zuständen oder Aktivitäten zusammenhängen. Diese Signale werden durch Sensoren aufgenommen und numerisch, methodisch analysiert und parametrisiert. Messbare Grössen können zum Beispiel diejenigen sein, die durch ein Elektrokardiogramm (EKG) von einem Menschen abgeleitet werden.

### Sensoren

Sensoren dienen dazu, die Signale (physiologische Parameter) zu erfassen und sie in elektrische Signale (in der Regel analog) umzuwandeln.

### Psychointeraktivität

Unter dem Begriff Psychointeraktivität ist eine Erweiterung des Begriffs der Interaktivität auf dem psychischen und mentalen Prozess zu verstehen. Die Psychointeraktivität kann zwischen Mensch und Mensch, aber auch zwischen Mensch und Computer und anderen Nutzern, beispielsweise in einem Netzwerk, stattfinden.

### Herzfrequenz-Variabilität

Die Herzfrequenz ist die Anzahl der Herzschläge pro Minute. Die Herzschlagfrequenz oder Herzfrequenz wird häufig mit dem Puls gleichgesetzt. Die Herzfrequenz unterliegt unterschiedlichen Einflüssen. Ein wesentlicher Teil des Regulativs erfolgt durch das autonome Nervensystem im Gleichgewicht von stimuliertem Sympathikus und dämpfendem Parasympathikus und zusätzlich weitere im Blut zirkulierende Hormone.

Der Begriff Herzfrequenz-Variabilität ist mit dem Begriff Herzratenvariabilität sowie der Abkürzung HRV identisch.

Je stärker der augenblickliche Anteil des Parasympathikus ist, desto ausgeprägter ist der Wert der Herzfrequenz-Variabilität. Wird der Körper in einen Stresszustand gebracht, dass heisst er soll aktiv werden, tritt der Parasympathikus zugunsten des Sympathikus zurück, die Herzschläge werden regelmässiger. Je besser diese beiden Anteile, Sympathikus und Parasympathikus, zusammen arbeiten, um so besser kann sich das Herz, wie auch der gesamte menschliche Organismus auf wechselnde Situationen einstellen und angemessen reagieren (Homöostase). Die Herzfrequenz-Variabilität beschreibt daher die Fähigkeit des Herzens, den zeitlichen Abstand von einem Herzschlag zum nächsten ständig neu anzupassen und sich so flexibel den ständig wechselnden Herausforderungen zu stellen. Eine ausgeprägte Herzfrequenz-Variabilität ist gegenüber einer Herzfrequenzstarre erstrebenswert. Folgende Handlungen und Tätigkeiten haben Einfluss auf die Herzfrequenz-Variabilität:
- vegetatives Nervensystem (Parasympathikus und Sympathikus)
- Atmung
- Lagewechsel (Liegen, Sitzen, Stehen)
- Tageszeit (zirkadianer Rhythmus)
- Lebensalter (hohe Werte bei Kindern, Abnahme mit zunehmendem Alter)
- interindividuelle Unterschiede (genetischer Einfluss)
- psychophysischer Stress, mentale Anspannung
- Erkrankungen (z. B. Abnahme bei Infekten)
- körperliche Belastung

Eine Variabilitätsanalyse der Herzfrequenz dient dazu, die Herzfrequenz-Änderungen über einen Zeitraum zu erfassen und für diagnostische und prognostische Zwecke nutzbar zu machen. Einzelne Komponenten dieser Variabilität weisen eine gewisse Regelmässigkeit auf und können mittels einer auf der diskreten Fourier-Transformation basierenden Methode quantifiziert werden. Hier lassen sich typische, empirisch festgelegte Frequenzbänder (ULF (0 - 3,3 mHz), VLF (3,3 mHz - 40 mHz), LF (40 mHz - 0,15 Hz) und HF (0,15 Hz - 0,4 Hz)) identifizieren, die mit physiologischen Prozessen wie Atmung (HF) und Aktivität der Blutdruckregulation (LF) in Verbindung gebracht werden.

Das zentrale Nervensystem in der Ausbildung eines vegetativen Nervensystems ergänzt die Steuerung lebenswichtiger Körperfunktionen. Das vegetative Nervensystem ist vom geistigen Willen eines Menschen weitgehend unbeeinflussbar und wird daher auch als autonomes Nervensystem bezeichnet. Es steuert die inneren Organe und Funktionen wie Atmung, Verdauung, Kreislauf und kontrolliert damit auch die Herzfunktionen. Das autonome Nervensystem hat zwei Elemente, den Sympathikus und den Parasympathikus, die sich in ihrer Funktionalität ergänzen beziehungsweise gegeneinander ausbalancieren: während der Sympathikus vornehmlich in Richtung einer Leistungssteigerung wirkt, arbeitet der Parasympathikus darauf hin, die Organe vor Überbeanspruchung zu bewahren und die Lebensvorgänge möglichst wirtschaftlich und ökonomisch zu gestalten. Die Balance der Funktionen des Sympathikus und des Parasympathikus sind daher wesentlich für das gesundheitliche Befinden des Menschen. Dies drückt sich im Bereich der Herz-Kreislauf-Funktionen unter anderem durch eine gewisse Variabilität der Herzfrequenz aus, die sich aus dem Zusammenwirken von Sympathikus (Beschleunigung) und Parasympathikus (Dämpfung) ergibt.

Die Analyse der Herzfrequenz-Variabilität erfolgt stets nach der festgestellten Messung. So kann in gewissen Abständen das gesundheitliche Befinden des Menschen durch die Analyse der Herzfrequenz-Variabilität festgestellt werden.

Die Variabilität der Herzfrequenz bezeichnet somit die Eigenschaft der Herzfrequenz des gesunden Menschen, sich ständig geringfügig zu ändern. Sie ist Ausdruck der diversen Regulationsmechanismen des Körpers, unter anderem hervorgerufen durch den Sympathikus und den Parasympathikus sowie durch Hormone. Dieses Regelsystem ist zur Erhaltung der Stabilität des Herz-Kreislaufsystems erforderlich.

### Elektrokardiogramm

Das Elektrokardiogramm (EKG) dient dazu, alle Aktivitäten der Herzmuskelfasern in elektrischer Form zu registrieren. Jede Pumpfunktion des Herzens wird durch eine entsprechende elektrische Erregung ausgeführt. Die entsprechenden Potenzialänderungen am Herzen, kann man über die Körperfläche mittels Sensoren ableiten und auf einer entsprechenden Zeitachse aufzeichnen. Die typische Darstellung eines Oberflächen-EKG erfolgt in der Abfolge P, PQ, ORS, ST, T, U, wobei die P-Welle der Vorhofverengung, der QRS-Komplex an der Kammererregung (Q erster negativer Ausschlag, R erster positiver Ausschlag und S negativer Ausschlag nach der R-Zacke), die T-Welle der Erregungsrückbildung der Kammer und die U-Welle einer nicht konstanten Erscheinung nach der T-Welle (durch Elektrolytstörung hervorgerufen) entspricht. Die entsprechenden Intervalle PQ bilden den Ausdruck der artrioventikulären Leistungszeit. Das QT-Intervall (auch QT-Zeit) zeichnet die intraventikuläre Erregungsdauer und die ST-Strecke entspricht der Null-Linie des Elektrokardiogramms.

Für die Herzfrequenz-Variabilität ist der Abstand des ersten positiven Ausschlages R des QRS-Komplexes bis zum nächsten ersten positiven Ausschlag des QRS-Komplexes massgeblich.

Dieser Intervall ändert sich über die entsprechende Zeit und wird Herzfrequenz-Variabilität genannt.

### MIDI-Signal

Unter dem Begriff MIDI-Signal ist eine digitale Schnittstelle für Musikinstrumente (musical instrument digital interface) zu verstehen. Es ist ein Datenübertragungsprotokoll, das die Kommunikation zwischen einem Personal Computer und verschiedenen Instrumenten ermöglicht. Das MIDI-Protokoll wird derzeit von vielen Soundkarten in Personal Computern unterstützt. Somit stellt das MIDI-Protokoll keine Klänge bereit, sondern besteht aus Befehlen zur Ansteuerung von Instrumenten oder der Soundkarte. Der entsprechende Befehl, der übermittelt wird, kann beispielsweise derart ausgestaltet sein, dass zum Beispiel dieser "Note-on" heisst (Schalte Ton an), "gebe die Anschlagstärke an" (velocity) und "Note-off" (Schalte Ton aus). Diese Steuerbefehle werden an einen Klangerzeuger, beispielsweise eine Soundkarte, Synthesizer oder eine entsprechend aufbereitete Klangbibliothek, die aus unterschiedlichen Instrumenten besteht, gesendet.

### Signalabfolgen

Unter Signalabfolge sind akustische oder visuelle Signale zu verstehen, die sequenziell ausgegeben werden. Diese Signale werden beispielsweise über MIDI-Steuersegmente aufgerufen.

### Signalsequenzen

Signalsequenzen sind mehrere Signalabfolgen als Cluster zusammengefasst. Signalsequenzen können beispielsweise mehrere Orchesterklänge sein. Es können Melodien entstehen.

### Stand der Technik

Die Aufnahme und die Darstellung der Herzfrequenz-Variabilität ist bereits aus dem Stand der Technik bekannt.

So ist aus der DE 102006002045 (DAILYCARE BIOMEDICAL INC (TW)) 03.08.2006 eine Vorrichtung zum Analysieren der Herzfrequenz-Variabilität bekannt. Die Analysevorrichtung ist integriert in ein Elektrokardiogramm-Signalmess und -verarbeitungsgerät. Die Vorrichtung verwendet die dem Elektrokardiogramm zugeordneten Messelektroden, wobei die Elektrokardiogrammsignale nach der Aufnahme verstärkt, gefiltert von Analog- in Digitalwerte umgewandelt werden. Eine Zeitbereichs- und Frequenzbereichsanalyse, beispielsweise eine Fourier-Transformation, dient dazu, die Berechnungen darstellbar zu machen. Dabei wird ein Berechnungsverfahren von der European Society of Cardiology und der North American Society of Pacing and Electro-physiology herangezogen. Über eine externe Darstellungseinrichtung, beispielsweise einem Monitor, kann das Ergebnis der Herzfrequenz-Variabilität ausgegeben werden.

Ein ähnlich vergleichbarer Weg ist in der Druckschrift CA 2490353 (ELLIOTT STEPHEN B (US)) 24.08.2005 dargestellt. Es handelt sich ebenfalls um eine Vorrichtung und ein Verfahren zum Vergleich des Atemrhythmusses mit der berechneten Herzfrequenz-Variabilität. Das System eignet sich dazu, die dargestellte Herzfrequenz-Variabilität dem Atemrhythmus anzupassen.

Aus der US 2006195038 (CARDIAC PACEMAKERS INC) 31.08.2006 ist ein Verfahren in Bezug auf die Herzfrequenz-Variabilität bekannt, das dazu geeignet ist, diese zu messen und diese auch entsprechend darzustellen. Die Darstellung erfolgt grafisch auf einem Monitor.

Aus der US 2004230130 (KUO TERRY B J (TW); YANG CHERYL C H (TW)) 18.11.2004 ist ebenfalls ein Verfahren und eine Vorrichtung zur Analyse der Herzfrequenz-Variabilität bekannt. Durch die Aufnahme der Signale eines Elektrokardiogramms, abgeleitet von einer Person, der Konvertierung von einem Analog- in ein Digitalsignal und Errechnung der Standardabweichung der "peaks" in Bezug auf die Zeitdauer und der entsprechenden Intervalle, kann eine aussagekräftige Information erstellt werden, die in Bezug auf die Balance zwischen dem Sympathikus und Parasympathikus entsprechende Informationen bereitstellt.

Aus der US 2001016694 (MEIER JAN H (DE); FOURNIE ERIC (DE)) 23.08.2001 ist ebenfalls ein Verfahren zur Berechnung der Herzfrequenz-Variabilität, abgeleitet von einem Elektrokardiogramm-Monitor dargestellt. Der Elektrokardiogramm-Monitor weist zusätzlich Berechnungshilfen, wie Fourier-Transformationen auf, um die Herzfrequenz-Variabilität grafisch darzustellen.

Bereits im Jahr 2000 sind von M. Ballora in einer Doctoral Thesis bei der International Conference on Auditory Display die Vertonung der Herzfrequenz-Variabilität dargestellt worden. Unter Zuhilfenahme eines bekannten Liedes sind Vektoren von der aufgenommenen Herzfrequenz-Variabilität entwickelt worden und jeder Vektor ist derart behandelt worden, als wäre er ein "Track" in einer Multi-Track-Musikaufnahme.

Aus der US 6662032 (INTERCURE LTD (IL)) 09.12.2003 ist ein Gerät zur Verbesserung der Gesundheit für den Nutzer bekannt, das einen ersten Sensor zur Aufnahme von physiologischen Daten aufweist. Ein zweiter Sensor ist dazu geeignet, das autonome Nervensystem des Nutzers aufzunehmen. Eine elektrische Schaltung sowie eine Vorrichtung sieht vor, die Daten der jeweiligen Sensoren zu empfangen, ein Ausgangsignal zu generieren und dem Nutzer eine Rückkopplung zu geben. In einer besonderen Ausführungsform ist vorgesehen, die physiologischen Daten direkt Tönen gleichzusetzen, so dass beispielsweise die Pulsation des Blutes akustisch über einen beispielsweise Klavierklang hörbar gemacht werden kann. Dies bedeutet, dass ein physiologischer Parameter derart von der elektrischen Schaltung umgesetzt wird, dass ein Ton und ein Rhythmus erzeugt wird. Alternativ und ergänzend dazu ist auch vorgesehen das so erhaltene und durch die elektrische Schaltung bearbeitete Signal durch ein MIDI-Interface auszugeben und so dem Nutzer eine entsprechende Rückkopplung zu geben. Es wurde festgestellt, dass durch diese Art der Rückkopplung die Atemfrequenz des jeweiligen Nutzers steuerbar wird.

Insbesondere ist die Wahl des Tones derart vorgegeben, dass der Nutzer zu einem idealen physiologischen Wert durch entsprechende Rückkopplungsmassnahmen geführt wird.

In der EP 0432152 (NEUROSONICS INC (US)) 19.06.1991 wird ein Gerät zum Umsetzen der Elektroenzephalogrammdaten (EEG) in Musik vorgeschlagen. Hieraus ist bekannt, dass die EEG-Aktivität, Muskel-Aktivität und andere physiologische Messungen durch Biofeedback modifizierbar sind. Die bis dahin bekannten konventionellen Biofeedback-Verfahren beinhalten das Umwandeln gewisser messbarer physiologischer Aktivitäten beziehungsweise Daten einer Person, in ein entsprechendes Rückkopplungssignal, welches einen hörbaren oder sichtbaren Reiz enthält. Damit wird dem Nutzer ein Rückkopplungssignal geliefert, das die tatsächlich gemessene physiologische Aktivität darstellt und möglicherweise auch einen Idealwert bereitstellt, den es zu erreichen gilt.

Insbesondere im EEG-Bereich ist es relativ schwierig, die Gehirnwellenaktivität tatsächlich aktiv zu beeinflussen. Daher kann das Biofeedback-Verfahren auch sehr ermüdend und langwierig, insbesondere auch langweilig sein, wenn nicht die entsprechende Qualität eines Ergebnisses erzielt wird.

Um dies zu vermeiden, wird eine Vorrichtung vorgeschlagen, welche die gemessenen Elektroströme durch einen Musiksynthesizer in Musik umwandelt. Somit wird ein musikalischer Kontext geschaffen, der als Rückkopplungsmassnahme in Bezug auf ein oder mehrere physiologische Daten, die durch Sensoren ermittelt und durch eine entsprechende Vorrichtung umgesetzt worden sind, erzeugt. So wird vorgeschlagen, die Töne beispielsweise durch Glockenklang oder ähnliche wirkende Instrumente auszubilden, damit der psychoakustischer Reiz für das Gehirn entsteht. Vorzugsweise haben die unterschiedlichen Sensoren unterschiedliche Klangtöne, so dass durch ein intensives Hören die jeweiligen Klangtöne separat durch physiologische Parameter, gesteuert durch den Nutzer selbst abgeändert werden können.

Es wird auch vorgeschlagen, dass den Tönen zusätzliche Musik beigefügt wird, um entsprechende interessante Effekte zu erzeugen. Beispielsweise kann man einem Signal Musik in Form eines "weissen Rauschens" hinzugeben bzw. die Musikeffekte damit "hintermalen", um dramatische donnerähnliche Effekte hervorzurufen, wenn durch das EEG-Signal gewisse Schwellenwerte überschritten werden. Somit stellt man sich vor, dass durch aktive Gehirnaktivität die einzelnen Töne aktiv geändert werden können.

Aus der US 7177672 (POLAR ELECTRO OY, FI) 13.02.2007 ist ein System zur Decodierung des Herzrhythmusses bekannt. Ist der Herzrhythmus durch einen Sensor entsprechend aufgenommen worden, so wird in einer entsprechenden Bibliothek Musik ausgewählt, die dem Herzrhythmus entspricht. Wird ein langsamerer Herzrhythmus angestrebt, so wird dem Nutzer aktiv eine Musik mit einem langsamen Rhythmus zugänglich gemacht, so dass durch äusserliche Einflussnahme aktiv der Herzrhythmus gesteuert werden kann. Die hierin dargestellte Vorrichtung dient dazu, Musik mit unterschiedlichen Takten bereitzustellen und diese dann abhängig von dem jweiligen tatsächlichen Herzschlagrhythmus oder von dem gewünschten Herzschlagrhythmus abzuspielen.

Ferner ist aus dem Stand der Technik bekannt, dass anhand einer Frequenzanalyse, deren Werte in Musik umgesetzt werden, Kurvenverläufe von Aktien über einen definierten Zeitraum hörbar gemacht werden.

Aus der US 7138575 B (ACCENTUS LLC, HANOVER, NH (US)) 28.05.2003 ist ein System und ein Verfahren bekannt, mittels dem Daten akustisch darstellbar gemacht werden. Eines sogenannte "Sonification Engine" übersetzt Daten, die auf einem Sever abgelegt sind und gibt sie als eine Tonfolge aus.

### Nachteile des Standes der Technik

Alle die vorgenannten Syteme oder Verfahren haben das gemeinsame Merkmal, dass der Benutzer zu einem idealen Wert - der durch den Programmierer vorgegeben und möglicherweise durch die Wissenschaft auch bestätigt ist - geführt wird. Sein eigenes Empfinden ist dabei nicht berücksichtigt. Dadurch ist die Ausgabe der Tonfolgen nicht wertfrei und nimmt auf das persönliche Empfinden des Benutzers keine Rücksicht.

Nachteilig an diesen genannten Verfahren ist, dass jeweils nur einzelne, stark an die errechneten Daten gekoppelte Töne ausgegeben werden, die lediglich die Variabilität der gemessenen Werte verdeutlichen, jedoch keine positive Stimulation des Verwenders ermöglichen. Dabei müssen die Daten aufwändig errechnet werden, was einen entsprechenden Zeitrahmen einnimmt.

Die Vorrichtung, die zur Berechnung verwendet werden und auch für die Darstellung eingesetzt werden, sind sehr komplex und eignen sich nicht, im alltäglichen Gebrauch eingesetzt zu werden.

Alle die zuvor genannten Vorrichtungen des Standes der Technik weisen die Eigenschaft auf, dass diese dem Nutzer Informationen, die physiologischen Ursprungs sind und mit den entsprechenden Vorrichtungen entsprechend akustisch umgesetzt werden, Informationen geben, die bewusst oder unbewusst dem Nutzer zu einem idealen vorgegebenen physiologischen Wert führen sollen.

### Aufgabe der Erfindung

Daher besteht die Aufgabe der Erfindung darin, eine Vorrichtung und ein Verfahren sowie eine Verwendung von physischen Daten darzustellen, mit deren Hilfe es möglich ist, beispielsweise Herzfrequenzvariabilität oder andere physische Daten des Nutzers unmittelbar in Echtzeit darzustellen und so die Möglichkeit eine mental erfahrbare wertfreie Rückkopplung zu geben.

### Lösung der Aufgabe

Die Lösung der erfindungsgemässen Aufgabe ergibt sich bezüglich der Vorrichtung aus den kennzeichnenden Merkmalen des Anspruchs 1, in Zusammenwirken mit den Merkmalen des zugehörigen Oberbegriffes. Die Lösung der erfindungsgemässen Aufgabe ergibt sich bezüglich des Verfahrens aus den kennzeichnenden Merkmalen des Anspruchs 10, in Zusammenwirken mit den Merkmalen des zugehörigen Oberbegriffes. Die Lösung der erfindungsgemässen Aufgabe ergibt sich bezüglich der Verwendung aus den Merkmalen des Anspruchs 17.

Weitere vorteilhafte Ausgestaltungen der Erfindung ergeben sich aus den jeweiligen Unteransprüchen.

### Vorteile der Erfindung

Eine der wesentlichen Unterschiede zu dem zuvor bereits beschriebenen Stand der Technik besteht bei der vorliegenden Erfindung darin, dass Vorrichtungen, die den Herzrhythmus in Form eines EKG aufnehmen, einen Herzklang erzeugen, der weit über einem Herzton (monoton) hinausgeht und direkt und wertfrei, höchstens nach musikalischen und künstlerischen Prinzipien dem Nutzer ein Feedback gibt, aber in der Form, dass durch dieses Feedback kein aktiver äusserer Regulationsprozess, sondern ein innerer Selbstregulationsprozess erzeugt wird, der die gewünschten Änderungen von sich aus herbeiführt.

Somit ist die Erfindung ein virtuelles Musikinstrument, das von den physiologischen Daten gesteuert wird und so wertfrei dem Nutzer ein Feedback gibt

Die erfindungsgemässe Vorrichtung dient zum Auswerten physiologischer Daten, insbesondere von Physiologische Daten (EKG-Daten) und umfasst dabei wenigstens einen Sensor, insbesondere EKG-Elektroden zur Aufnahme der EKG-Daten, eine Betriebssoftware zur Auswertung der EKG-Daten und zum Zuordnen der ausgewerteten EKG-Daten zu einem Steuersignal zur Ansteuerung einer Klangbibliothek sowie einer Ausgabeeinheit. Vorzugsweise wird hierfür ein MIDI-Signal verwendet.

Aus dem QRS-Komplex der EKG-Daten wird hierbei durch die Betriebssoftware die R-Spitze erfasst und das Zeitintervall zwischen zwei aufeinander folgenden R-Spitzen (auch NN genannt) ermittelt. Die so gewonnenen Daten werden mittels eines Bandfilters bearbeitet und spezifischen Frequenzbereichen innerhalb von Frequenzbänder (ULF (0 - 3,3 mHz), VLF (3,3 mHz - 40 mHz), LF (40 mHz - 0,15 Hz) und HF (0,15 Hz - 0,4 Hz)) zugeordnet. Die derart aufbereiteten Daten werden vorzugsweise an eine MIDI-Einheit weitergegeben, die dem jeweiligen Signal einen bestimmten Klang zuordnet und diesen über eine Ausgabeeinheit, beispielsweise einen Synthesizer mit angeschlossenem Lautsprecher, wiedergibt. Die erfindungsgemässe Vorrichtung ist dadurch gekennzeichnet, dass sie eine Speichereinheit für eine Vielzahl akustischer und/oder visueller Signalabfolgen, vorzugsweise in der Ausbildung von Klangbibliotheken, aufweist und die Zuordnung wenigstens einer Signalabfolge zu den aufbereiteten Daten vorgesehen ist. Es werden somit nicht mehr nur einzelne, in ihrer Aussage eher schwache Töne, sondern mehr oder weniger harmonische Klang- und/oder Farbabfolgen ausgegeben. Die Vorrichtung erlaubt es also den aus aufbereiteten physischen Daten (beispielweise EKG - Daten) ableitbaren, physischen oder psychischen Zustand eines Verwenders akustisch und/oder visuell zu verdeutlichen.

Den aufbereiteten physischen Daten wird beispielsweise bei ausgeglichener Herzfrequenz und stabilem Zustand des Verwenders eine ruhigere und harmonischere Klangfolge zugeordnet als den physischen Daten die bei gestressten oder kranken Verwenden ermittelt werden. Hier böte sich beispielsweise eine gehetztere, weniger ausgeglichene Klangfolge an, um dem Verwender seinen Zustand eindringlich zu vermitteln. Deutlicher wird es, wenn die Ansteuerung für die Ausgabe von Klangfolgen einer Orchesterbibliothek verwendet wird.

In einer besonders bevorzugten Ausführungsform ist die Vorrichtung so ausgestaltet, dass eine Verbindung der akustischen Signalabfolgen zu einer Sequenz erfolgt. Aus dieser Sequenz ergibt sich dann eine polyphone Melodie, die für den jeweiligen Wertebereich der aufbereiteten physischen Daten charakteristische Schwingungsanteile aufweist.

Um die Melodie weiter zu charakterisieren und um ein harmonischeres und ansprechenderes Klangbild zu erzeugen, weist die Sequenz bevorzugt eine Variation hinsichtlich Lautstärke und/oder Rhythmus der Signalabfolgen auf. Die somit aus den Herztönen abgeleitete, polyphone Melodie macht dem Verwender ebenso wie einem begleitenden Betreuer den psychischen und/oder physischen Zustand bewusst und es kann nachfolgend mit entsprechender Unterstützung erreicht werden, eine optimale Funktion von Sympathikus und Parasympathikus einzustellen. Die Übungen, die zum Erreichen dieser optimalen Funktion führen, übt der Verwender unter Anleitung ein und gelangt so zu einer der optimalen Funktion zugeordneten, charakteristischen Melodie. Dies geschieht jedoch völlig wertfrei und ohne Angabe eines idealen Wertes. Die Anpassung erfolgt ausschliesslich nach dem Wohlbefinden des Nutzers, der bestrebt ist, ein harmonisches Gleichgewicht für sich persönlich zu finden.

In besonders vorteilhafter Ausgestaltung ist vorgesehen, dass die Zuordnung in Echtzeit erfolgt. So erlaubt eine entsprechend ausgestattete Vorrichtung, dass während der Ableitung der physischen Werte eine Zusammenstellung der Sequenzen durchgeführt wird. So können beispielsweise auftretende Frequenzstörungen oder-Sprünge in die Gesamtsequenz beziehungsweise Melodie eingefügt werden, um so als Grundlage für Änderungen in der Signalausgabe zu dienen. Eine nachträgliche Zuordnung der Signalabfolgen beziehungsweise die nachträgliche Bearbeitung der Sequenzen erlaubt es dem Betreuer eine harmonische und dem Verwender positiv beeinflussende Melodie zu generieren, die keine negativen Spitzentöne aufweist, da diese den angestrebten einheitlichen Grundcharakter der Melodie stören würden. Daneben ist es auch möglich, bereits die Eingangssignale und die daraus berechneten Daten in anderer Weise zu analysieren, zum Beispiel Standardabweichungen, Korrelationen oder Variationen zu berechnen und deren Tendenzen zu analysieren. Dazu verfügt die Betriebssoftware über einen Funktionsbereich, in dem der Betreuer die für die Signalanalyse zu verwendeten Algorithmen frei definieren kann.

Um eine weitere Verbesserung der ausgegebenen Signalabfolgen und der daraus generierbaren Melodien zu erreichen, weisen die akustischen Signalabfolgen günstigerweise eine Variation ihrer Klangattribute, insbesondere hinsichtlich Klangart, Klangfarbe, Tonhöhe und/oder Lautstärke auf. Somit kann die Melodie weiter individualisiert, harmonischer gestaltet und für den Verwender angenehmer ausgebildet werden.

Um einen für den Verwender möglichst vertrauten Gesamteindruck zu erzeugen, sind die akustischen Signalabfolgen in einer vorteilhaften Ausgestaltung der erfindungsgemässen Vorrichtung aus Aufnahmen wenigstens eines Musikinstrumentes und/oder einer Stimme generiert. Es wird somit vermieden, dass die generierte Melodie eine elektronische und daher eher steril anmutende Klangfärbung erhält. Der Klang eines Instrumentes und/oder einer menschlichen Stimme wird als besonders beruhigend beziehungsweise stimulierend empfunden und trägt somit zu einer weiteren Verbesserung des Allgemeinzustandes bei.

In einer Weiterbildung der Vorrichtung ist es denkbar, dass eine zusätzliche Speichereinheit für die den ausgewerteten physischen Daten zugewiesenen Signalabfolgen und/oder Sequenzen vorgesehen ist. Die während einer Anwendung der Vorrichtung generierten Melodien können dann beispielsweise auf einem Tonträger gespeichert werden und dem Verwender als Grundlage für eigene Übungen dienen. Bei diesen eigenen Übungen hört der Verwender die Melodien wieder an und versucht sich in eine ähnliche Balance zu bringen, wie er es in Begleitung des Betreuers erreicht hat. Das Abhören der Melodien stimuliert dabei ein unbewusstes Erinnern an den in der Sitzung erreichten Gleichgewichtszustand und stellt so ein effizientes Hilfsmittel für die eigene Übung dar.

Es ist weiterhin besonders vorteilhaft, wenn die visuellen Signalabfolgen als Zuordnungen zu einer Farbtafel, und hierbei bevorzugt über die RGB-Werte der Farbtafel, darstellbar sind.

Günstigerweise ist die Zuordnung zudem über den Helligkeitswert steuerbar ausgebildet.

Farben können die verschiedensten Reaktionen im Menschen auslösen, da die Farben mit verschiedenen Zuständen oder Gegenständen (zum Beispiel rot mit Wärme, blau mit Kälte) assoziiert werden. Auf den Betrachter wirken beispielsweise Signalabfolgen in hellen Farben anregender als überwiegend dunkle Signalabfolgen, die eher dämpfende Wirkung aufweisen. Der gezielte Einsatz farbiger Signalabfolgen, auch in Verbindung mit entsprechenden akustischen Signalabfolgen, kann den Verwender unbewusst beeinflussen und dessen Allgemeinzustand weiter verbessern beziehungsweise eine noch effektivere Durchführung der Übungen bewirken. Zudem gibt die Möglichkeit zur visuellen Darstellung der Signalabfolgen auch hörbehinderten Verwender die Möglichkeit, die Vorrichtung zu verwenden. Eventuell kann in diesem Fall eine Kombination einer visuellen und einer rhythmischen Ausgabe der Signalabfolge erfolgen, die dann besonders gut von dieser Nutzergruppe wahrnehmbar ist.

In einer weiteren vorteilhaften Ausführungsform der vorgestellten Erfindung ist vorgesehen, dass die Vorrichtung in ein tragbares Datenverarbeitungsgerät integriert ist. Diese Ausgestaltung gibt dem Verwender die Möglichkeit, flexibel und ortsunabhängig seinen Gesundheitszustand beziehungsweise dessen Verbesserung oder Verschlechterung zu überprüfen und bei einer Warnung durch die Vorrichtung selbstständig durch Übungen auf die ermittelten Werte zu reagieren. Die Vorrichtung kann dabei beispielsweise in ein PDA-Gerät integriert werden, das entsprechende Anschlüsse für die Sensoren und eine Auswertesoftware für die ermittelten Herzfrequenzdaten aufweist.

Zusätzlich wird es als günstig angesehen, wenn das tragbare Gerät eine Schnittstelle zur Datenübertragung aufweist. Hierdurch hat der Verwender die Möglichkeit, die gemessenen und aufbereiteten Werte beziehungsweise die daraus generierte Signalabfolge entweder direkt an seinen Betreuer zu übertragen, so dass dieser die Möglichkeit zur Fernüberwachung erhält, zum anderen ist somit auch eine Übertragung von unterwegs generierten Signalabfolgen an einen stationären Personal Computer möglich, um die Daten, Signalabfolgen oder Sequenzen weiter zu bearbeiten oder erneut abzuspielen. Darüber kann auch der Gesundheitszustand überwacht beziehungsweise können Veränderungen über längere Zeitintervalle hinweg beobachtet werden. Über die Datenschnittstelle hat im Gegenzug selbstverständlich auch der Betreuer die Möglichkeit, das Übungsprogramm des Verwenders durch Veränderung der Messparameter, wie beispielsweise die Anpassung der statistischen Auswertung der ermittelten Herzfrequenz oder über Wechsel des Frequenzbandes des Bandfilters, zu beeinflussen.

Eine gattungsgemässe Vorrichtung wird dadurch weitergebildet, dass wenigstens ein zusätzlicher Sensor zur Aufnahme physiologischer Daten, insbesondere der Körpertemperatur, des Hautleitwertes und/oder der Atemfrequenz vorgesehen ist.

Der zusätzliche Sensor wird neben den Sensoren zur Erfassung der physischen Daten, insbesondere EKG-Elektroden, am Verwender appliziert und über Zuleitungen mit der Vorrichtung verbunden. Die Messungen dieses Sensors werden dann mit den aus der Herzfrequenz gewonnenen Daten korreliert, um ein noch genaueres Zustandsbild des Verwenders zu erhalten und um die ausgegebene Signalabfolge noch besser auf dieses Zustandsbild abstimmen zu können.

Aus den mit Hilfe der Vorrichtung gewonnenen Daten kann somit nicht nur eine gezielte Beeinflussung des Verwenders erfolgen, es kann aus den ermittelten Daten auch eine Prognose für die weitere Entwicklung des Zustandes abgeleitet werden, um die zukünftigen Übungen konkret auf die zu erwartende Zustandsänderung abzustimmen.

Ein erfindungsgemässes Verfahren zur Zuordnung von Signalabfolgen zu physiologische Daten umfasst mehrere Schritte. Zunächst wird eine Ermittlung von physiologische Daten durchgeführt. Dies erfolgt durch nicht-invasive Applikation von EKG-Elektroden auf der Haut des Verwenders. Nach Aufnahme der Daten werden diese mit Hilfe einer Betriebssoftware verarbeitet und dabei aus dem QRS-Komplex des EKG-Signals die R-Spitze erfasst und das Intervall zwischen zwei aufeinander folgenden R-Spitzen ermittelt. Die gewonnenen Daten werden mittels eines Bandfilters bearbeitet und spezifischen Frequenzbereichen innerhalb der Frequenzbänder (ULF (0 - 3,3 mHz), VLF (3,3 mHz - 40 mHz), LF (40 mHz - 0,15 Hz) und HF (0,15 Hz - 0,4 Hz)) zugeordnet. Die so ausgewerteten physiologische Daten werden an eine MIDI-Einheit weitergegeben, die dem jeweiligen Signal einen bestimmten Ton zuordnet und das Signal über eine Ausgabeeinheit, beispielsweise einen Synthesizer mit angeschlossenem Lautsprechersystem wiedergibt. Das Verfahren ist dadurch gekennzeichnet, dass den aufbereiteten physiologische Daten wenigstens eine akustische und/oder visuelle Signalabfolge zugeordnet wird.

Die Signalabfolgen werden in einer vorteilhaften Weiterbildung des erfindungsgemässen Verfahrens zu einer Sequenz verbunden, sodass eine Melodie entsteht, die charakteristische Elemente enthält und somit über die aufbereiteten Physiologische Daten den Zustand des Verwenders widerspiegelt. Durch entsprechende Übungen, beispielsweise Atemübungen, Entspannung, progressive Muskelrelaxion, Ausdauertraining, autogenes Training et cetera kann der Verwender Verhaltensweisen einüben, mittels derer er in der Lage ist, zunächst unter fachlicher Anleitung und später selbstständig seine Herzfrequenz beziehungsweise die daraus abgeleiteten Variabilitätswerte zu beeinflussen.

Von besonderem Vorteil ist es, wenn dabei die Zuordnung der Signalabfolgen in Echtzeit oder nachträglich erfolgt. Die Verarbeitung der Daten und die Ausgabe der Signalabfolge in Echtzeit ermöglicht es dem Verwender eine sofortige Aussage über seinen aktuellen Zustand zu erhalten. Im Rahmen einer nachträglichen Auswertung kann eine noch genauere Analyse der Daten durchgeführt werden, um so beispielsweise durch Veränderung des Bandfilters oder die Veränderung statistischer Auswertungsparameter bereits kleinste Schwankungen in der Herzfrequenz beziehungsweise in den daraus abgeleiteten Werten sichtbar zu machen. Im Zuge der nachträglichen Datenauswertung kann auch eine bessere Abstimmung der zugeordneten Signalabfolgen durchgeführt werden, was zu einem insgesamt harmonischer empfundenen Gesamtbild führt.

Ebenfalls ist es denkbar, dass das Klangbild der ausgegebenen Signalabfolgen oder der aus diesen zusammengesetzten Klangsequenzen weiter verbessert werden kann, in dem die akustischen Signalabfolgen in Klang, Klangfarbe, Tonhöhe und/oder Lautstärke variieren.

Vorteilhafterweise ist die akustische Signalabfolge zudem aus Aufnahmen wenigstens eines Musikinstrumentes und/oder einer Stimme generiert, da im Vergleich zu künstlich generierten Tönen, Klänge echter Instrumente beziehungsweise die Singstimme eines Menschen oftmals als angenehmer empfunden werden.

Bei der visuellen Darstellung der ermittelten Daten wird als günstig angesehen, wenn die visuellen Signalabfolgen als Zuordnungen zu einer Farbtafel, vorteilhafterweise über die Zuordnungen zu den RGB-Werten der Farbtafel, dargestellt werden. Denkbar ist zudem, dass die Zuordnungen über den Helligkeitswert gesteuert werden.

Um eine noch genauere Darstellung auch geringster Schwankungen der ermittelten Werte darstellen zu können, weist die aus den Signalabfolgen gebildete Sequenz bevorzugt eine Variation hinsichtlich Lautstärke und Rhythmus der Signalabfolgen auf.

Im Rahmen einer fachlichen Betreuung wird dem Verwender eine aus EKG-Daten abgeleitete, polyphone Melodie hörbar und damit auch bewusst gemacht. Der Verwender wird dann auf eine für ihn optimale Funktion von Sympathikus und Parasympathikus eingestellt, und er übt anschliessend ein, den Zustand der Sitzung selbstständig wieder zu erreichen. Eine weitere Ausgestaltung des erfindungsgemässen Verfahrens sieht daher vor, dass zur nachträglichen Auswertung oder zum erneuten Abhören der generierten Sequenz oder Melodie, die den jeweiligen ausgewerteten EKG-Daten zugewiesene Signalabfolge und/oder die aus Signalabfolgen zusammengestellte Sequenz in einer Speichereinheit gespeichert und/oder auf Datenträger ausgegeben wird. Die für die optimale Funktion charakteristische Melodie kann dem Verwender somit als Grundlage für eigene Übungen dienen, da bei den eigenen Übungen die Melodien wieder angehört und versucht wird, sich in eine ähnliche Balance zu bringen, wie sie in Begleitung des Betreuers erreicht wurde. Das Abhören der Melodien stimuliert dabei ein unbewusstes Erinnern an den in der Sitzung erreichten Gleichgewichtszustand und stellt so ein effizientes Hilfsmittel für die eigene Übung dar.

In einer Weiterbildung ist es denkbar, dass die den ausgewerteten EKG-Daten zugewiesene Signalabfolge und/oder die Sequenzen über eine Datenübertragungsschnittstelle weitergegeben werden. Dabei ist es möglich, dass die gemessenen und aufbereiteten Werte beziehungsweise die daraus generierte Signalabfolge beispielsweise über GSM, W-LAN oder vergleichbare Datenübertragungen an einen Betreuer zu übertragen, so dass dieser die Möglichkeit zur Ferndiagnose erhält. Weiterhin ist es auch denkbar, dass eine Übertragung von unterwegs generierten Signalabfolgen an einen stationären Personal Computer erfolgt, um die Daten, Signalabfolgen oder Sequenzen weiter zu bearbeiten, auszuwerten oder erneut abzuspielen. Somit kann der Gesundheitszustand beziehungsweise dessen Veränderung über längere Zeitintervalle hinweg beobachtet werden. Über die Datenschnittstelle hat im Gegenzug selbstverständlich auch der Betreuer die Möglichkeit, das Übungsprogramm des Verwenders durch Veränderung der Messparameter, wie beispielsweise die Veränderung der statistischen Auswertung der ermittelten Herzfrequenz oder über Wechsel des Frequenzbandes des Bandfilters, in Echtzeit zu beeinflussen.

Das Verfahren wird dadurch weitergebildet, dass weitere physiologische Daten, insbesondere die Körpertemperatur, der Hautleitwert und/oder die Atemfrequenz aufgenommen werden und eine Korrelation der aufgenommenen Daten mit den ausgewerteten Physiologische Daten erfolgt.

Aus diesen zusätzlichen Daten kann somit ein noch genaueres Bild des Verwenders generiert werden und die ausgegebene Signalabfolge noch besser auf dessen Zustand abgestimmt werden, da hierbei auch Änderungen im Allgemeinzustand berücksichtigt werden, die sich nicht unmittelbar in einer Änderung der Herzfrequenz äussern.

Mit Hilfe der so gewonnenen Daten kann nicht nur eine gezielte Beeinflussung des Verwenders erfolgen, es kann auch eine Prognose für die weitere Entwicklung des Zustandes durchgeführt werden, um die zukünftigen Übungen konkret auf die zu erwartende Zustandsänderung abzustimmen.

Erfindungsgemäss ist vorgesehen, die von einer eingangs beschriebenen Vorrichtung aufgenommenen beziehungsweise in einem entsprechenden Verfahren ausgewerteten physiologische Daten zur musikalischen Darstellung der physiologische Daten zu verwenden. Dabei wird es als besonders vorteilhaft erachtet, wenn die musikalische Darstellung in Form von polyphonen Orchesterstimmen erfolgt, um eine möglichst harmonische und vom Verwender als angenehm empfundene Melodie zu erzeugen.

Die erfindungsgemässe Vorrichtung, sowie das zugeordnete Verfahren, können auch verwendet werden, um die Komposition eines Musikstückes durchzuführen. Das so komponierte Musikstück kann dabei individuell für den Verwender erstellt werden.

Darüber hinaus ist es ebenfalls denkbar, dass die physiologische Daten verwendet werden, um daraus Werte zur visuellen Darstellung der Herzfrequenz beziehungsweise deren Variabilität darzustellen. Es lassen sich somit Bild oder Farbabfolgen generieren, die die jeweilige Situation des Verwenders widerspiegeln. Die erstellten Farbabfolgen können dann im Rahmen einer Farbtherapie oder zur stimmungsabhängigen Beleuchtung, beispielsweise von Räumen, herangezogen werden. Die visuelle Darstellung erfolgt vorteilhafterweise in Form von Zuordnungen zu einer Farbtafel.

Die aus der abgeleiteten Herzfrequenz generierten Signalabfolgen und/oder Sequenzen können in weiterer Ausgestaltung auch dahingehend verwendet werden, als dass eine Verknüpfung der musikalischen, also akustischen, und der visuellen Darstellung erfolgt. Bestimmten Klängen oder Melodien werden dann abgestimmte Abfolgen von Farbwechseln zugeordnet, so dass ein optisch-akustischer Gesamteindruck entsteht. Dieser ist auf die unbewusste, aktuelle Verfassung des Verwenders abgestimmt und kann durch gezielte Übungen verändert werden.

Eine bevorzugte Ausführungsform der beschriebenen Vorrichtung, des Verfahrens sowie der Verwendung besteht darin, diese als Biofeedback-Einrichtung zu nutzen. Aufgrund der psychoregulativen Möglichkeiten des Nutzers, kann im Echtzeitmodus das dargestellte Ergebnis entweder auf dem Monitor oder auch in Form der charakteristischen Töne beziehungsweise der charakteristischen aufgenommenen Musik beeinflusst werden. Da der Nutzer/Verwender im Gegensatz zum Stand der Technik nicht mehr darauf angewiesen ist, unmittelbar auf ein Monitor oder auf einen Ausdruck zu schauen und sich schon alleine auf diesen zu konzentrieren, kann er mit geschlossenen Augen über die akustische Wahrnehmung bestrebt sein, ein harmonisches Klanggefühl zu erreichen, in dem er Parameter, wie Atmung, Lagewechsel, mentale Anspannung, körperliche Belastung oder Ähnliches ändert. Eine Psychointeraktivität kann entstehen. In seinem völlig entspanntem Zustand erhält der Nutzer ein harmonisches, akustisches Klanggefüge, das charakteristisch ist für seinen speziellen "Herzklang". Dieser Herzklang kann vorteilhafterweise aufgenommen werden und in unterschiedlichen Datenformaten abspeicherbar sein. Es entsteht dadurch eine "Herzmusik".

Dies bringt wiederum den Vorteil mit sich, dass die Person, insbesondere in gestressten Situationen, die Möglichkeit hat, diesen harmonischen, klangvollen und für die Person speziellen akustischen Klang zu hören, so dass sich selbstständig ein anderes wohlwollenderes, wohlfühlenderes, wohltuendes Gleichgewicht einstellt.

Daher kann das erfinderische Biofeedback-Verfahren, die Vorrichtung und auch die entsprechende Verwendung zum einen dafür eingesetzt werden, um interaktiv in Echtzeitmodus unterschiedliche Parameter, die die paraphysiologischen Gegebenheiten des Körpers steuern, auszuprobieren, wie diese auf die Herzfrequenz-Variabilität Einwirkung haben, um so sein optimales Gleichgewicht zu finden. Zum anderen kann es dafür eingesetzt werden, das einmal gefundene, optimierte und für die Person entsprechend angepasste Gleichgewicht, immer wieder durch höheren derart auf die Person wirken zu lassen, dass sich dieses Gleichgewicht dann auch einstellt.

Weitere vorteilhafte Ausgestaltungen gehen aus der nachfolgenden Beschreibung, den Ansprüchen sowie den Zeichnungen hervor.

### Zeichnungen

Es zeigen:

- Fig. 1: einen schematischen Aufbau der Vorrichtung;
- Fig. 2: eine Übersicht über die Verfahrensschritte des erfindunsgemässen Verfahrens;
- Fig. 3: ein Beispiel eine Echtzeitdarstellung eine Pulsrate (obere Linie) und eines Quotienten aus LF/HF (untere Linie).

### Beschreibung der Ausführungsbeispiele

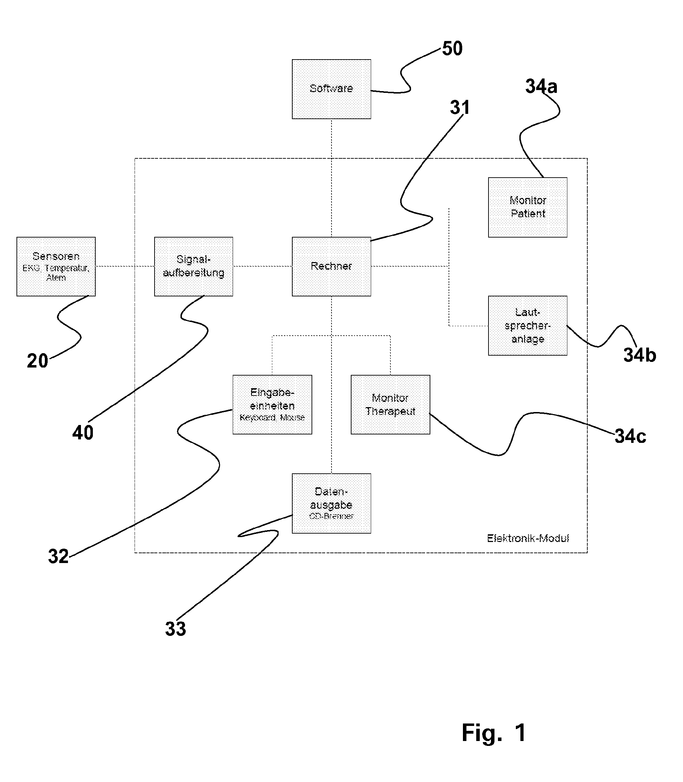

In Figur 1 wird schematisch der Aufbau der erfindungsgemässen Vorrichtung dargestellt. Die Vorrichtung 10 eignet sich zur Auswertung von Herzfrequenzdaten. Zur Aufnahme der Herzfrequenz sowie weiterer physiologischer Daten umfasst die Vorrichtung Sensoren 20, wie beispielsweise EKG-Elektroden zur Erfassung physiologischer Daten, insbesondere EKG-Daten, Sensoren für die Leitfähigkeit der Haut und Sensoren zur Überwachung der Atemfrequenz. Die Sensoren 20 werden in Form von einzelnen Elektroden oder eines Sensormoduls (z. Bsp. Brustgurt) am Verwender appliziert und über Zuleitungen und eine entsprechende Schnittstelle mit dem Elektronik-Modul 30 verbunden.

Das Elektronik-Modul 30 umfasst einen leistungsfähigen Desktopbeziehungsweise vergleichbaren Mobil-Rechner 31, oder in einer einfacheren Ausführungsform einen tragbaren PDA oder ein vergleichbar ausgerüstetes Mobiltelefon. Im Elektronik-Modul 30 werden im Zuge der Signalaufbereitung 40 die Spannungen, die aus den Elektroden der Sensoren 20 kommen, mit einer sehr hochohmigen Eingangsstufe verstärkt, gefiltert und mit 16-bit bei einer relativ hohen Abtastrate, (>5 ksps) digitalisiert. Die digitalen Signale werden im Rechner 31 des Elektronik-Moduls 30 von der Betriebssoftware 50 verarbeitet.

Über Eingabeeinheiten 32, wie beispielsweise Tastatur, Maus oder ein sonstiges Eingabegerät zur Steuerung grafischer Benutzeroberflächen, kann der Verwender die Datenauswertung beeinflussen. Ebenfalls im Elektronik-Modul 30 erfolgt die Datenaufzeichnung. Dazu verfügt das Elektronik-Modul 30 beispielsweise über einen CD- oder DVD-Brenner 33 oder über Anschlussmöglichkeiten für weitere Speichermedien wie beispielsweise USB-Speicherstifte et cetera. Die in Klänge und Farben umgesetzten EKG-Daten sowie die damit gegebenenfalls korrelierten physiologischen Daten werden auf einem oder mehreren der Vorrichtung 10 zugeordneten Ausgabegeräten 34 a, b, c dargestellt. So kann verwenderseitig ein Monitor 34a und eine Lautsprecheranlage 34b vorgesehen werden, wohingegen seitens des Betreuers lediglich ein Monitor 34c zur Ausgabe der Werte sowie der aufbereiteten Daten verwendet wird.

Der in Figur 1 gezeigte Aufbau der Vorrichtung 10 eignet sich für den fachlich qualifizierten Einsatz. Daneben ist auch eine Version der Vorrichtung für den Gebrauch durch den nicht geschulten Verwender denkbar. Während das allgemeine Funktionsprinzip beider Vorrichtungen 10 gleich ist, unterscheiden sich diese in der Bedienung und den Zugriffsmöglichkeiten. Die Vorrichtung 10 zur Nutzung durch den nicht geschulten Verwender weist ebenfalls die oben genannten Elementen (Sensoren 20, Elektronik-Modul 30, Betriebssoftware 50) auf, das Elektronik-Modul 30 und die Funktionalität der Betriebssoftware 50 sind hierbei jedoch wesentlich vereinfacht. Das Elektronik-Modul 30 beinhaltet eine Schnittstelle für die Sensoren 20 und eine weitere Schnittstelle zu einer externen Lautsprecheranlage 34b. Als Elektronik-Modul 30 kann ein PDA oder einfaches Notebook verwendet werden. Die Funktionalität ist dann auf die visuelle Darstellung der Herzfrequenzkurve, der daraus ermittelten Herzfrequenz sowie ihrer Variabilität und auf die Anzeige der spektralen Leistungsdichte in definierten Frequenzbändern (zum Beispiel: LF (40 mHz - 0,15 Hz) und HF (0,15 - 0,4 Hz)) beschränkt. Die akustische Wiedergabe ist auf einige vorgegebene Klangbilder eingeschränkt, die vom Verwender ausgewählt werden können und über die an den PDA oder das einfache Notebook angeschlossenen Lautsprecheranlage 34b wiedergegeben werden. Wird ein PDA oder ein Notebook mit Anschluss an das Kommunikationsnetz verwendet (GPRS, WLAN), kann optional eine direkte Übertragung der Daten an den Betreuer erfolgen.

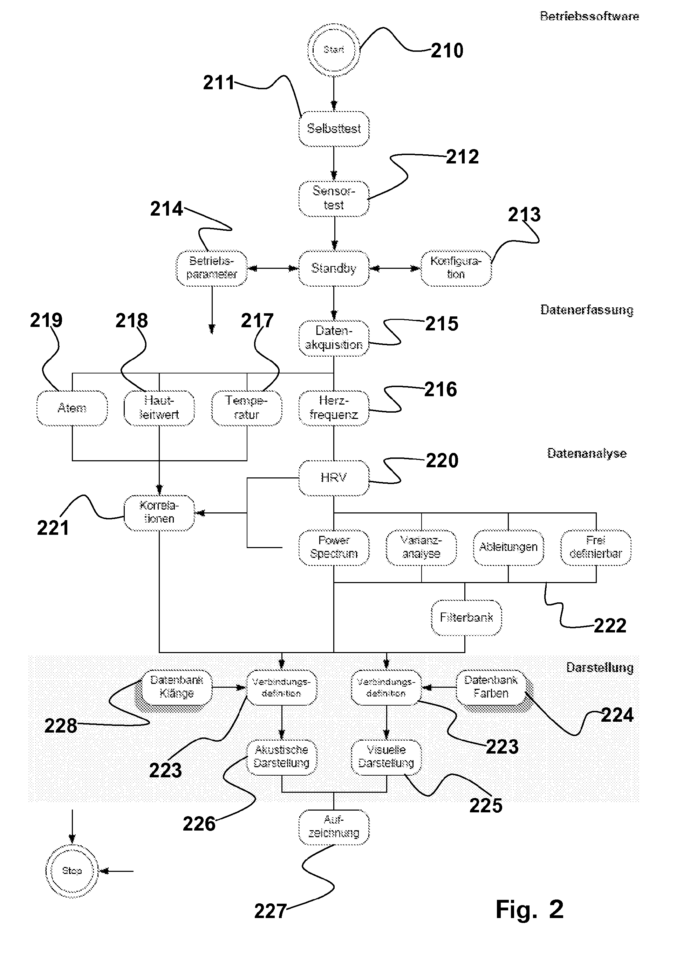

Figur 2 zeigt eine Übersicht über die erfindungsgemässen Verfahrensschritte. So erfolgt nach Start 210 der Vorrichtung und der Betriebssoftware 50 zunächst ein Selbsttest 211 der Vorrichtung 10 und der Betriebssoftware 50 sowie eine Funktionsprüfung 212 der Sensoren 20. Nach einem entsprechenden Konfigurationsschritt 213 und der Einstellung der Betriebsparameter 214 erfolgt die Datenaquisition 215 über die Sensoren 20. Hierbei wird vor allem die Herzfrequenz 216 aufgezeichnet, optional können zusätzlich Werte für die Körpertemperatur 217, den Hautleitwert 218 sowie die Atemfrequenz 219 ermittelt und der Betriebssoftware 50 zwecks Korrelation 221 mit der Herzfrequenz 217 zur Verfügung gestellt werden.

Aus den Eingangssignalen werden die Herzfrequenzkurve und die Standardableitungen gebildet. Über eine einfache Signalanalyse wird aus der Herzfrequenzkurve die Herzfrequenz 216 berechnet, sowie deren Änderung und ihre erste und zweite Ableitung der Variabilitätsfunktion 220. Aus der Herzfrequenzkurve 216 wird über die Fourier-Transformation der zeitlichen Autokorrelationsfunktion des Signals das Leistungsdichtespektrum errechnet. Alternativ kann die Signalanalyse auch mit Hilfe von Wavelets erfolgen. Das Leistungsdichtespektrum wird im Bereich von 0 bis 100 Hz berechnet. Dieses Leistungsdichtespektrum kann direkt angezeigt oder auch nach seinen Frequenzinhalten analysiert werden. Dazu können durch den Verwender Frequenzintervalle definiert werden, für welche dann der entsprechende Leistungsanteil aus dem Leistungsdichtespektrum berechnet wird. Die Bandbreite der Frequenzintervalle ist frei wählbar und kann zwischen 0,1 mHz und 1 Hz liegen. Die Lage der Frequenzintervalle kann innerhalb des gesamten Frequenzbereichs frei gewählt werden, wobei die Wahl der Mittenfrequenz der Frequenzintervalle mit einer Auflösung von 0,02 mHz erfolgt. Als Basis sind die in der Kardiologie empirisch ermittelten Frequenzbänder ULF (0 - 3,3 mHz), VLF (3,3 mHz - 40 mHz), LF (40 mHz - 0,15 Hz) und HF (0,15 Hz - 0,4 Hz) vordefiniert.

Daneben ist es in einem zusätzlichen Verfahrensschritt 222 möglich, die Eingangssignale und die daraus berechneten Daten auch in anderer Weise zu analysieren. Hierzu können zum Beispiel Standardabweichungen, Korrelationen oder Variationen berechnet werden, um deren Tendenzen zu analysieren. Dazu bietet das Verfahren dem Verwender die Möglichkeit, die für die Signalanalyse zu verwendeten Algorithmen frei zu definieren.

Zusätzlich können alle durch die Software errechneten Daten durch den Nutzer angewählt und am Bildschirm in Echtzeit dargestellt werden. Der Nutzer kann dabei die Darstellung und die Gestaltung des Bildschirms innerhalb gewisser Grenzen frei definieren. Der Bildschirm für den Betreuer wird dabei entsprechend anders konfiguriert sein als der für den Verwender.

Aus technischer Sicht sind drei verschiedene Arten von Umsetzung der physischen Daten in Klangfolgen denkbar:

A. Ein Digitalfilter (im gesamten Bereich LF-HF) filtert in Abhängigkeit der Frequenz aus den errechneten Daten beispielsweise Amplitudenwerte heraus, die anschliessend an einen MIDI-Player mit einer konstanten Klang, beispielsweise einem virtuellen Sänger, übergeben werden. B. Verschiedene Digitalfilter filtern in verschiedenen Frequenzbereichen beispielsweise Amplituden heraus, deren Werte an einen MIDI-Player mit verschiedenen Klangbereichen (beispielsweise unterschiedliche Chöre) übergeben werden. C. Verschiedene Digitalfilter filtern in verschiedenen Frequenzbereichen beispielsweise Amplituden und andere Werte heraus, die an den MIDI-Player (Controller) übergeben werden, und war zur Ansteuerung der Tonlage, der Lautstärke oder anderer klanglich bestimmenden Eigenschaften.

Die Ergebnisse dieser mathematischen Signalanalyse können zum einen visualisiert, zum anderen aber auch hörbar gemacht werden. Bei der Visualisierung können Amplituden, Phasen, Frequenzen oder auch Spektren durch entsprechende Zuordnungen zu einer Farbtafel dargestellt werden. Diese Zuordnungen werden über die RGB-Werte der Farbtafel gemacht, die zusätzlich noch über den Helligkeitswert gesteuert werden können. Über die dem Verwender dargestellte Farbgebung der Analyseergebnisse und ihren Wechsel lassen sich die Wirkungen durch die Nutzung der Erkenntnisse der Farbenpsychologie wirkungsvoll unterstützen. Nach Verbindungsdefinition 223 wird die entsprechende visuelle Darstellung 225 aus einer Datenbank "Farben" 224 ausgewählt und anschliessend dargestellt. Zusätzlich kann die visuelle Darstellung 225 zusammen mit der akustischen Darstellung 226 zur Aufzeichnung 227 weitergegeben werden.

Einen weiteren wesentlichen Verfahrensschritt stellt die Möglichkeit zur akustischen Vermittlung der Analyseergebnisse dar. Damit kann ein weiterer Zugang zum Unbewussten erschlossen werden. Den ausgewerteten Messergebnissen können in Echtzeit verschiedene Klänge, Klangfarben, Tonhöhen, Lautstärken oder andere Klangeffekte aus einer Datenbank "Klänge" 228 flexibel zugeordnet werden. Aus dieser Zuordnung ergeben sich polyphone Melodien mit komplexem Aufbau, aus denen sich auf die psychische Disposition des Verwenders abgestimmte und auf ein Therapieziel hin optimierte musikalische Sequenzen ergeben. Diese Klänge werden aus Aufnahmen von realen Musikinstrumenten in hoher Qualität generiert, um einen für den Verwender angenehmen Klangeindruck zu erzeugen.

Werden Digitalfilter oder Bandpass-Filter für die Analyse der physischen Daten, beispielsweise der EKG Daten eingesetzt und diese berechneten Daten wiederum zur Steuerung eines MIDI-Controllers eingesetzt, so konnte folgendes festgestellt werden:

A. Das Intervall R-R bei EKG Daten ist kohärent mit der Atemfrequenz.

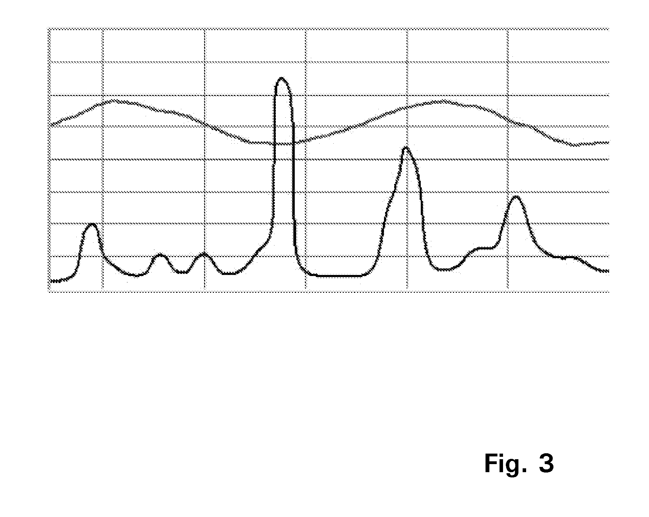

B. Der Amplituden-Ausgang des Filters über den vollständigen Bereich LF-HF kann zur Ansteuerung der Lautstärke verwendet werden (Fig. 3).

C. Ein polyphones Ensemble kann glissandoartig modulierend dargestellt werden, wenn aus der Analyse heraus das Herzfrequenzvariabilitäts-Frequenzspektrum herangezogen wird.

Darüber hinaus können den verschiedenen Klängen und Melodien auch dynamisch sich verändernde Visualisierungen zugeordnet werden, mit denen die ablaufenden Vorgänge optisch unterstützt werden können.

Bei der visuellen Darstellung 225, wie auch bei der akustischen Darstellung 226, ist es möglich, den Verwender über Farbgebungen oder Klangbilder auf das Optimum der physiologischen Funktionen hin zu steuern. Ist die Funktion über dem Optimum, wird das Display zum Beispiel in Rot-Tönen gehalten, ist die Funktion unter dem Optimum, herrschen Blau-Töne vor, ist es optimal, werden Grün-Töne vorherrschen.

Somit ist das wesentliche Element der erfindungsgemässen Vorrichtung die Möglichkeit zur akustischen Vermittlung von Analyseergebnissen, die aus physischen Daten, direkt abgeleitet von dem Körper des Benutzers, bestehen. Dadurch erschliesst sich ein weiterer Zugang in das Unterbewusstsein des Nutzers. Diesen Analyseergebnissen werden dann in Echtzeit, abhängig von unterschiedlichen Berechnungsverfahren, wie Fast Fourier-Transformation, Standard-Abweichungen oder ähnliches, Klänge, Klangfarben, Instrumente mit oder ohne Klangeffekte zugeordnet, wobei sich aus dem dann entstehenden Klangbild eine polyphone Melodie ergibt. Der Aufbau dieser Melodie kann sehr komplex sein.

### Bezugszeichenliste

- 10: Vorrichtung
- 20: Sensoren
- 30: Elektronik-Modul
- 31: Mobil-Rechner

- 32: Eingabehilfe
- 33: CD- oder DVD-Brenner
- 34a: Monitor
- 34b: Lautsprecheranlage
- 34c: Monitor
- 40: Signalaufbereitung
- 50: Betriebssoftware
- 210: Start
- 211: Selbsttest
- 212: Funktionsprüfung
- 213: Konfigurationsschritt
- 214: Betriebsparameter
- 215: Datenaquisition
- 216: Herzfrequenz
- 217: Körpertemperatur
- 218: Hautleitwert
- 219: Atemfrequenz
- 220: Variabilitätsfunktion
- 221: Korrelation
- 222: Verfahrensschritt
- 223: Verbindungsdefinition
- 224: Datenbank "Farben"
- 225: visuelle Darstellung
- 226: akustische Darstellung
- 227: Aufzeichnung
- 228: Datenbank "Klänge"

## Patentansprüche

1. Vorrichtung zum Auswerten von physiologischen Parametern, insbesondere von physiologische Daten, umfassend:
- wenigstens einen Sensor (20), insbesondere Elektrokardiogramm-Elektrode, zur Aufnahme der physiologische Daten;
- eine Betriebssoftware (50) zur Auswertung der physiologische Daten und zur Zuordnung der ausgewerteten physiologische Daten zu mindestens einem Steuer-Signal, und
- wenigstens eine Ausgabeeinheit (34 a,b,c) für das Steuer-Signal,
**dadurch gekennzeichnet, dass** die Vorrichtung (10) eine Speichereinheit für eine Vielzahl akustischer und/oder visueller Signalabfolgen, insbesondere in der Ausbildung einer Klangbibliothek aufweist, wobei die Zuordnung wenigstens einer akustischen und/oder visuellen Signalabfolge zu den ausgewerteten physiologische Daten in Echtzeit vorgesehen ist.

2. Vorrichtung nach Anspruch 1, **dadurch gekennzeichnet, dass** die akustischen Signalabfolgen zu einer Sequenz verbindbar sind.

3. Vorrichtung nach Anspruch 2, **dadurch gekennzeichnet, dass** die Sequenz eine Variation hinsichtlich Lautstärke und/oder Rhythmus der Signalabfolgen aufweist.

4. Vorrichtung nach einem der Ansprüche 1 bis 3, **dadurch gekennzeichnet, dass** die Zuordnung in Echtzeit oder nachträglich erfolgt.

5. Vorrichtung nach einem der vorhergehenden Ansprüche, **dadurch gekennzeichnet, dass** die akustischen Signalabfolgen eine Variation hinsichtlich ihrer Klangattribute, insbesondere Klangart, Klangfarbe, Tonhöhe und/oder Lautstärke aufweisen.

6. Vorrichtung nach einem der vorhergehenden Ansprüche, **dadurch gekennzeichnet, dass** die akustischen Signalabfolgen aus Aufnahmen wenigstens eines Musikinstrumentes und/oder einer Stimme generiert sind.

7. Vorrichtung nach einem der vorhergehenden Ansprüche, **dadurch gekennzeichnet, dass** die visuellen Signalabfolgen als Zuordnungen zu einer Farbtafel darstellbar sind.

8. Vorrichtung nach einem der vorhergehenden Ansprüche, **dadurch gekennzeichnet, dass** wenigstens ein zusätzlicher Sensor (20) zur Aufnahme physiologischer Daten, insbesondere Körpertemperatur (217), Hautleitwert (218) und/oder Atemfrequenz (219) vorgesehen ist und wobei eine Korrelation (221) der aufgenommenen Daten mit den ausgewerteten Physiologische Daten vorgesehen ist.

9. Vorrichtung nach einem der vorhergehenden Ansprüche, **dadurch gekennzeichnet, dass** die Vorrichtung (10) in ein tragbares Datenverarbeitungsgerät (31) integriert ist.

10. Verfahren zur Zuordnung von Signalabfolgen zu physiologische Daten, umfassend die Schritte:
- Ermittlung der physiologische Daten (215);
- Auswertung der physiologische Daten;
- Zuordnen der ausgewerteten physiologische Daten zu einem MIDI-Signal, und
- Ausgabe eines akustischen Signals (226),
**dadurch gekennzeichnet, dass** den ausgewerteten physiologische Daten wenigstens eine akustische und/oder visuelle Signalabfolge zugeordnet wird.

11. Verfahren nach Anspruch 10, **dadurch gekennzeichnet, dass** die Zuordnung in Echtzeit oder nachträglich erfolgt.

12. Verfahren nach einem der Ansprüche 10 oder 10, **dadurch gekennzeichnet, dass** die akustischen Signalabfolgen in Klang, Klangfarbe, Tonhöhe und/oder Lautstärke variieren.

13. Verfahren nach einem der Ansprüche 10 bis 12, **dadurch gekennzeichnet,**
**dass** die akustische Signalabfolge aus Aufnahmen wenigstens eines Musikinstrumentes und/oder einer Stimme generiert wird.

14. Verfahren nach einem der Ansprüche 10 bis 13, **dadurch gekennzeichnet,**
**dass** die visuellen Signalabfolgen als Zuordnungen zu einer Farbtafel dargestellt werden.

15. Verwendung von physiologische Daten zur musikalischen Darstellung dieser Physiologische Daten.

16. Verwendung nach Anspruch 15, **dadurch gekennzeichnet, dass** die musikalische Darstellung in Form von polyphonen Orchesterstimmen erfolgt.

17. Verwendung von physiologische Daten zur musikalischen Darstellung dieser physiologische Daten in Echtzeit als Biofeedback-Vorrichtung.
